# EUROPEAN PATENT SPECIFICATION

(11) **EP 2 210 573 B1**
(45) Date of publication and mention of the grant of the patent: **23.12.2015**
(21) Application number: 08841842.1
(22) Date of filing: 16.10.2008
(51) Int. Cl.: A61F 2/915, A61F 2/82, A61L 27/54

(54) **NESTED STENT IN BODY LUMEN**
EINGEBETTETER STENT IN EINEM KÖRPERLUMEN
ENDOPROTHÈSE IMBRIQUÉE DANS UNE LUMIÈRE CORPORELLE

(30) Priority: 17.10.2007 CN 200710047122
(43) Date of publication of application: 28.07.2010
(73) Proprietor: Shanghai MicroPort Medical (Group) Co., Ltd., Shanghai 201203 (CN)
(72) Inventor: XIE, Zhiyong, Shanghai 201203 (CN); LUO, Qiyi, Shanghai 201203 (CN); LI, Yu, Shanghai 201203 (CN); LI, Jianjun, Shanghai 201203 (CN); WANG, Changchun, Shanghai 201203 (CN); WANG, Sen, Shanghai 201203 (CN); HAO, Xia, Shanghai 201203 (CN); TANG, Zhirong, Shanghai 201203 (CN)
(74) Representative: Dennemeyer & Associates S.A.
(86) International application number: PCT/CN2008/001745
(87) International publication number: WO 2009/052703

(56) References cited:
- EP-A1- 1 466 634
- WO-A1-03/047463
- CN-A- 1 605 366
- CN-A- 1 709 210
- CN-A- 1 993 155
- CN-Y- 2 920 185
- CN-Y- 201 135 513
- US-A1- 2004 225 350
- US-A1- 2006 224 229
- US-A1- 2006 224 234
- US-B1- 6 758 859

## Description

### FIELD OF INVENTION

The present invention relates to a minimally invasive medical device, in particular relates to a nested stent used in a body lumen.

### BACKGROUND OF THE INVENTION

In recent years, with the development of intervention technology, PCI (percutaneons coronary intervention) has been more and more widely used in curing cardiopathy caused by Coronary Atherosclerosis. In this process, an expandable metal stent is implanted at the dysfunctional site of a blood vessel, so as to expand the stenosed sites of the blood vessel for curing stenosis. However, after the metal stent is implanted, it is inclined to cause restenosis due to the features such as the elastic modulus of the metal being inconsistent with those of the blood vessel. It is reported that restenosis occurred after implanting the metal stent is about 30 %.

Currently, a stent is usually designed in a straight strut type, the diameter of which is larger after pressed and held with a balloon, and the traversing ability for a complex site of lesion is poor. Also, after expanding, the stent with a straight strut type is not able to contact a larger vascular wall region and effectively cover more plaque regions, which increases the probability of vascular restenosis.

Furthermore, there is now a stent with a drug coating, which may decrease the probability of vascular restenosis. In general, the stent with a drug coating comprises a stent and a drug coating thereon. The drug coating usually is a polymer coating in which drug is contained. The drug coating is applied to the outer surface of a metal strut of the stent, which can achieve a local release of drug at the lesion site, so as to effectively inhibit occurrence of vascular restenosis under the condition that the concentration in local tissue is

high while the whole body concentration is very low. However, due to limitation of outer surface area of the stent metal strut, the amount of drug-loading is low, thereby not meeting the clinical needs.

WO 03/047463 A1 relates to a stent designed for the delivery of therapeutic substance or other agents.

To overcome at least one of the above shortcomings, the present invention provides a stent, which can travel across complex lesion sites and/or carry a large amount of drug.

### SUMMARY OF THE INVENTION

The technical problem to be solved by the present invention is to overcome at least one of the shortcomings of the prior art. There is provided a nested stent used in body lumen to overcome deficiencies such as poor traversing ability for complex lesion sites and a high probability of vascular restenosis.

According to the embodiments, there is provided a nested stent used in body lumen, which is formed of a plurality of stent struts connecting each other, characterized in that adjacent stent struts are nested each other when the stent is pressed and held.

The stent struts are provided with concavo-convex and interlace-arranged segments thereon, and the segments on the adjacent stent struts are nested each other in a meshing shape when the stent is pressed and held.

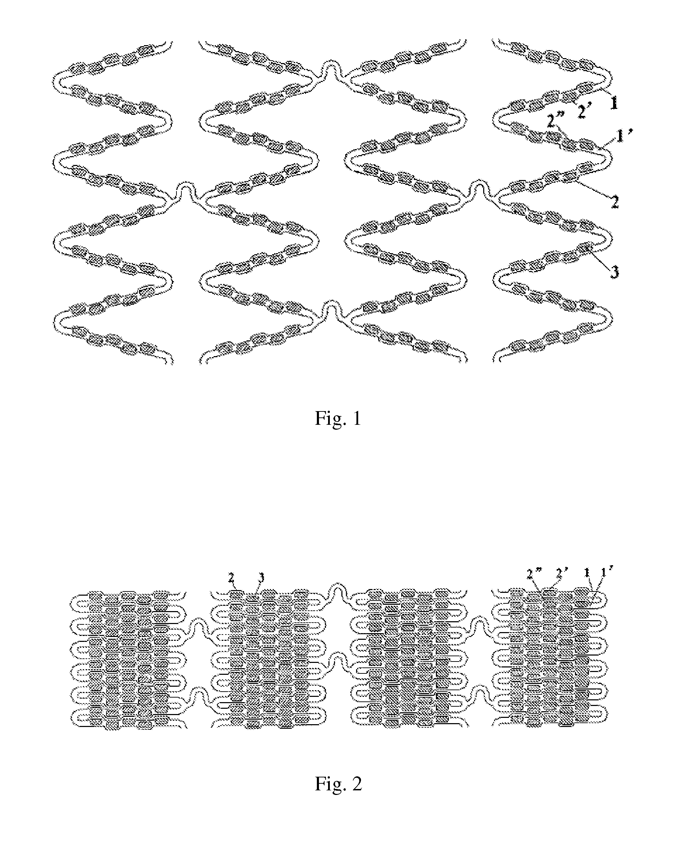

The said adjacent stent struts is shown as stent struts 1 and 1' in Fig.1, wherein the numeral symbols 1 and 1' denote two adjacent stent struts.

It can be seen that, in the present invention, the adjacent stent struts nest each other in a meshing shape when the stent is pressed and held, and a smaller pressed/held diameter may be obtained, so that the stent can easily travel across and access to the stenosed and complex lesion sites. Compared to a conventional stent with a straight strut type, the nested stent of the present invention can contact a wider region of vascular lesion sites after expanding, and effectively function as a good support for the stenosed blood vessel. The present invention further provides a nested stent used in a body lumen with a drug-loading slot, the drug-loading slot is arranged at a place of the stent struts, on which a force had very little effect and there is no stress, so as to ensure that the stent has no loss in structural strength. Since the surface area of the slots is larger, a larger amount of drug can be loaded. During the expanding and deformation of the stent, drug-loading slots may not be deformed, so that the drug in the slots can not be peeled off and broken away from the slots. In the present invention, the drug-loading slot is used as a carrier for drug, which is different from the conventional polymer coating. After the drug releases completely, the stent functions as a naked stent, so that some side effects such as delayed endothelialization, late stent malapposition and inflammation caused by residue of the conventional polymer coating can be eliminated.

### BRIEF DESCRIPTION OF THE DRAWINGS

Figure 1 is a local plane structural schematic view illustrating of a stent used in a body lumen after expanding according to a first embodiment of the present invention.
Figure 2 is a local plane structural schematic view of the stent used in a body lumen in Figure 1 in a pressed and held state.
Figure 3 is a local plane structural schematic view illustrating of a stent used in a body lumen after expanding according to a second embodiment of the present invention.
Figure 4 is a local plane structural schematic view of the stent used in a body lumen in Figure 3 in a pressed and held state.
Figure 5 is a cross-sectional enlarged structural schematic view illustrating a rectangular slot according to an embodiment of the present invention.
Figure 6 is a cross-sectional enlarged structural schematic view illustrating a trapezoid slot according to an embodiment of the present invention.
Figure 7 is a cross-sectional enlarged structural schematic view illustrating a U-shape slot according to an embodiment of the present invention.
Figure 8 is a cross-sectional enlarged structural schematic view illustrating a V-shape slot according to an embodiment of the present invention.

### DESCRIPTION OF THE PREFERRED EMBODIMENTS

Specific examples of the present invention will be further illustrated with reference to the drawings.

As shown in Figure 1-4, a nested stent used in a body lumen of the invention is formed with a plurality of stent struts connecting each other, and the adjacent stent struts 1 are nested each other when the stent is pressed and held.

The stent struts 1 are provided with concavo-convex and interlace-arranged segments 2 thereon, the segments 2 on the adjacent stent struts 1 are nested to each other in a meshing shape when the stent is pressed and held.

An embodiment of the invention is shown as in Fig.1, in which the corresponding segments on the adjacent stent struts are interlace-arranged in a concavo-convex shape. That is, segment 2' at a position on stent strut 1 is concave, while the segment 2" at a corresponding position on the adjacent stent strut 1' is convex. The concave of segment 2' and the convex of segment 2" on the two adjacent stent struts 1, 1' are in a complementary configuration, so that the two adjacent struts can be nested to each other after being pressed and held, as shown in Figure 2.

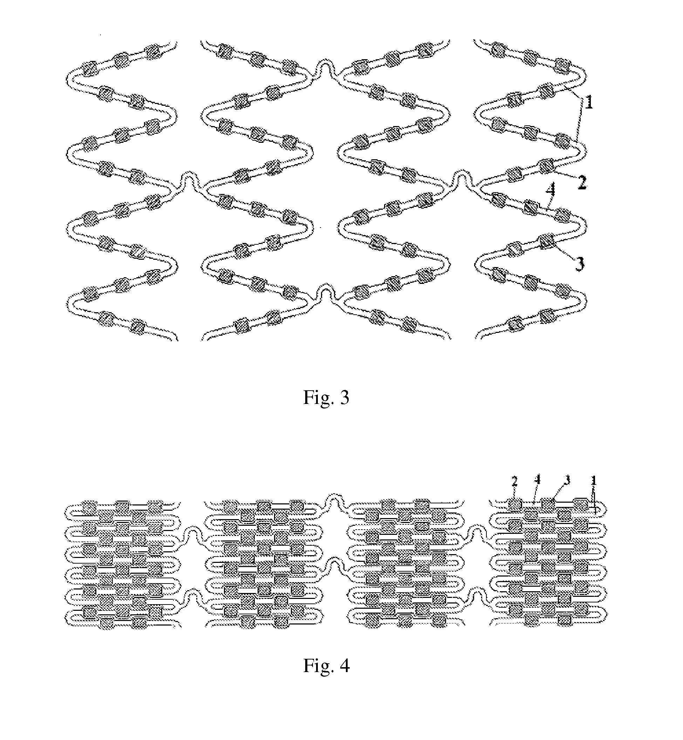

Another embodiment of the invention is shown as in Fig. 3 and Fig. 4, in which segments 2 are provided on each stent strut 1, and between two adjacent segments 2 on each stent strut 1 is a concave segment 4. The width of the segment 2 is slightly larger than the width of the basal body of the stent struts 1 such as the concave segment 4. The segment 2 is arranged on a stent strut 1 at positions corresponding to the concave segment 4 on an adjacent stent strut 1, and the number of the segment 2 on a stent strut is odd while the number of the segment 2 on an adjacent stent strut is even. That is, the segments 2 on the adjacent stent struts are odd-even arranged.

Further, the stent struts can be provided with a drug-loading slot 3 thereon. The drug-loading slot 3 is distributed on the outer surface of the stent, and the drug-loading slot may be either penetrating or non-penetrating, that is, the drug-loading slot may (or may not) penetrate through the strut. The drug-loading slots may be distributed on the segments 2 of stent struts 1.

The drug-loading slot 3 may be a single dead hole or groove, or may also be combination of dead holes and grooves.

The shape of the drug-loading slot 3 may be the shape of circular, rectangular, oval, rhombus and the like, or other irregular shapes, which are distributed on the stent struts 1 and spaced from each other at a certain interval. The number of the drug-loading slot 3 may be 0 to 10, preferable 2 to 6, on one stent strut.

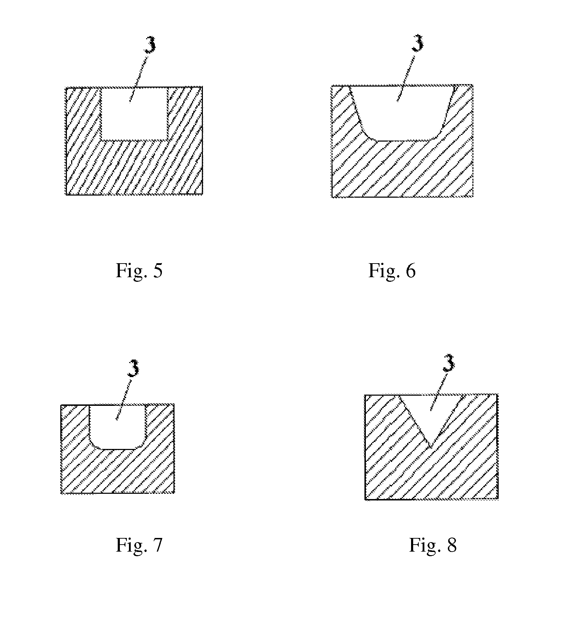

The cross-sectional shape of the drug-loading slot 3 may be rectangular (Figure 5), trapezoid with a longer top side and a shorter bottom side (Figure 6), U-shape (Figure 7), and V-shape (Figure 8), or other irregular shapes. Wherein, the width of the drug-loading slot 3 is in the range of 0.021 mm to 0.085 mm, and preferable in the range of 0.030 to 0.070 mm, and most preferable 0.060 mm; the depth of the drug-loading slot 3 is in the range of 0.021 to 0.080 mm, preferable the depth is in the range of 0.021 to 0.055 mm, most preferable 0.035 mm. The total cross-section area of all the drug-loading slots 3 on one stent strut 1 is 10% to 50% of the total cross-section area of the said one stent strut 1. In order to ensure a sufficient strength and support force for the stent struts 1, the cross-section area of all the drug-loading slots 3 on one stent strut 1 is 20% to 40% of the total cross-section area of the said one stent strut 1 in which case the width of the drug-loading slot 3 should be no less than 0.05 mm, and the depth of the drug-loading slot 3 should be no less than 0.02 mm.

The stent used in a body lumen of the present invention may be made of metal or non-metal material, and the metal may be a material with good biocompatibility such as cobalt-based alloy, stainless steel, and magnesium alloy and the like. Non-penetrating slots or holes, or penetrating slots or holes can be formed by using laser etching technology at the outer surface of stent metal struts.

## Claims

1. A nested stent used in a body lumen, which is formed of a plurality of stent struts connecting each other, wherein adjacent stent struts (1) are nested to each other when the stent is pressed and held, the stent struts (1) being provided with drug-loading slots (3) thereon, **characterized in that** the total cross-section area of all the drug-loading slots (3) on one stent strut (1) is 20% to 40% of the total cross-section area of the said one stent strut (1) and wherein the widths of a drug-loading slot (3) should be no less than 0.05 mm, and the depth of a drug-loading slot (3) should be no less than 0.02 mm.

2. The stent used in a body lumen according to claim 1, wherein the stent struts (1) are provided with concavo-convex and interlace-arranged segments (2) thereon, and the segments on the adjacent stent struts (1) are nested each other in a meshing shape when the stent is pressed and held.

3. The stent used in a body lumen according to claim 1, wherein the stent struts (1) are provided with segments (2) thereon, the segments (2) on adjacent stent struts 1 are odd-even arranged, and the width of the segments (2) is slightly larger than that of the basal body of the stent struts (1).

4. The stent used in a body lumen according to claim 1, wherein the stent struts (1) are provided with a drug-loading slot (3) thereon, the drug-loading slot (3) is distributed on the outer surface of the stent, and the drug-loading slot is either penetrating or non-penetrating.

5. The stent used in a body lumen according to claim 2, wherein the segments (2) of the stent struts (1) are provided with a drug-loading slot (3) thereon, and the drug-loading slot (3) is distributed on the outer surface of the stent.

6. The stent used in a body lumen according to either claim 4 or 5, wherein the shape of the drug-loading slot (3) is circular, rectangular, oval, rhombus, or other irregular shapes.

7. The stent used in a body lumen according to claim 4, wherein the number of drug-loading slots (3) on one stent strut (1) is 0 to 10, and the drug-loading slots (3) are distributed on the one stent strut (1) and spaced from each other if the number of the drug-loading slot (3) is more than one.

8. The stent used in a body lumen according to either claim 4 or 5, wherein the cross-sectional shape of the drug-loading slot (3) is rectangular, trapezoid with a longer top side and a shorter bottom side, U-shape, V-shape, or other irregular shapes.

9. The stent used in a body lumen according to either claim 4 or 5, wherein the width of the drug-loading slot (3) is in the range of 0.021 mm to 0.085 mm.

10. The stent used in a body lumen according to either claim 4 or 5, wherein the width of the drug-loading slot (3) is in the range of 0.030 to 0.070 mm.

11. The stent used in a body lumen according to either claim 4 or 5, wherein the depth of the drug-loading slot (3) is in the range of 0.021 mm to 0.080 mm.

## Patentansprüche

1. Verschachtelter Stent zum Einsatz in einem Körperlumen, der aus einer Mehrzahl miteinander verbundener Stentstreben besteht, wobei angrenzende Stentstreben (1) miteinander verschachtelt werden, wenn der Stent gedrückt und festgehalten wird, wobei die Stentstreben (1) mit Wirkstoffbeladungsschlitzen (3) darauf versehen sind, **dadurch gekennzeichnet, dass** die gesamte Querschnittfläche aller Wirkstoffbeladungsschlitze (3) an einer Stentstrebe (1) 20 - 40 % der gesamten Querschnittfläche der einen Stentstrebe (1) beträgt, und wobei die Breiten eines Wirkstoffbeladungsschlitzes (3) nicht weniger als 0,05 mm betragen sollten, und die Tiefe eines Wirkstoffbeladungsschlitzes (3) nicht weniger als 0,02 mm betragen sollte.

2. Stent zum Einsatz in einem Körperlumen nach Anspruch 1, wobei die Stentstreben (1) mit konkav-konvexen und verschachtelt angeordneten Segmenten (2) darauf versehen sind, und die Segmente an den angrenzenden Stentstreben (1) maschenförmig miteinander verschachtelt werden, wenn der Stent gedrückt und festgehalten wird.

3. Stent zum Einsatz in einem Körperlumen nach Anspruch 1, wobei die Stentstreben (1) mit Segmenten (2) darauf versehen sind, und die Segmente (2) an den angrenzenden Stentstreben (1) in einer Ungerade-Gerade-Konfiguration angeordnet sind und die Breite der Segmente (2) etwas größer ist als die des Grundkörpers der Stentstreben (1).

4. Stent zum Einsatz in eine Körperlumen nach Anspruch 1, wobei die Stentstreben (1) mit einem Wirkstoffbeladungsschlitz (3) darauf versehen sind, wobei der Schlitz (3) auf der Außenfläche des Stents verteilt ist und der Wirkstoffbeladungsschlitz entweder durchdringend oder nicht durchdringend ist.

5. Stent zum Einsatz in eine Körperlumen nach Anspruch 2, wobei die Segmente (2) der Stentstreben (1) mit einem Wirkstoffbeladungsschlitz (3) darauf versehen sind, und der Wirkstoffbeladungsschlitz (3) auf der Außenfläche des Stents verteilt ist.

6. Stent zum Einsatz in einem Körperlumen nach Anspruch 4 oder 5, wobei die Form des Wirkstoffbeladungsschlitzes (3) kreisförmig, rechteckig, oval, rhomboid ist oder eine andere unregelmäßige Form aufweist.

7. Stent zum Einsatz in einem Körperlumen nach Anspruch 4, wobei die Anzahl der Wirkstoffbeladungsschlitze (3) an einer Stentstrebe (1) 0 bis 10 beträgt, und die Wirkstoffbeladungsschlitze (3) auf der einen Stentstrebe (1) verteilt und voneinander beabstandet sind, wenn die Anzahl der Wirkstoffbeladungsschlitze (3) mehr als 1 beträgt.

8. Stent zum Einsatz in einem Körperlumen nach Anspruch 4 oder 5, wobei die Querschnittsform des Wirkstoffbeladungsschlitzes (3) rechteckig, trapezförmig mit einer längeren Ober- und einer kürzeren Unterseite, U-förmig, V-förmg ist, oder andere unregelmäßige Formen aufweist.

9. Stent zum Einsatz in einem Körperlumen nach Anspruch 4 oder 5, wobei die Breite des Wirkstoffbeladungsschlitzes (3) im Bereich von 0,021 bis 0,085 mm liegt.

10. Stent zum Einsatz in einem Körperlumen nach Anspruch 4 oder 5, wobei die Breite des Wirkstoffbeladungsschlitzes (3) im Bereich von 0,030 bis 0,070 mm liegt.

11. Stent zum Einsatz in einem Körperlumen nach Anspruch 4 oder 5, wobei die Tiefe des Wirkstoffbeladungsschlitzes (3) im Bereich von 0,021 bis 0.080 mm liegt.

## Revendications

1. Stent imbriqué utilisé dans une lumière corporelle, qui est formé avec une pluralité d'entretoises de stent se raccordant entre elles, dans lequel les entretoises de stent (1) adjacentes sont imbriquées les unes dans les autres lorsque le stent est comprimé et maintenu, les entretoises de stent (1) étant prévues avec des fentes de charge de médicament (3) sur ces dernières, **caractérisé en ce que** la surface transversale totale de toute les fentes de charge de médicament (3) sur une entretoise de stent (1) représente de 20% à 40% de la surface transversale totale de ladite une entretoise de stent (1) et dans lequel les largeurs d'une fente de charge de médicament (3) ne doivent pas être inférieures à 0,05 mm, et la profondeur d'une fente de charge de médicament (3) ne doit pas être inférieure à 0,02 mm.

2. Stent utilisé dans une lumière corporelle selon la revendication 1, dans lequel les entretoises de stent (1) sont prévues avec des segments concavo-convexes (2) et agencés en étant entrelacés sur ces dernières, et les segments sur les entretoises de stent (1) adjacentes sont imbriqués les uns dans les autres selon une forme d'engrènement lorsque le stent est comprimé et maintenu.

3. Stent utilisé dans une lumière corporelle selon la revendication 1, dans lequel les entretoises de stent (1) sont prévues avec des segments (2) sur ces dernières, les segments (2) sur les entretoises de stent (1) adjacentes sont agencés de manière paire-impaire, et la largeur des segments (2) est légèrement plus grande que celle du corps de base des entretoises de stent (1).

4. Stent utilisé dans une lumière corporelle selon la revendication 1, dans lequel les entretoises de stent (1) sont prévues avec une fente de charge de médicament (3) sur ces dernières, la fente de charge de médicament (3) est répartie sur la surface externe du stent et la fente de charge de médicament est pénétrante ou non pénétrante.

5. Stent utilisé dans une lumière corporelle selon la revendication 2, dans lequel les segments (2) des entretoises de stent (1) sont prévus avec une fente de charge de médicament (3) sur ces derniers et la fente de charge de médicament (3) est répartie sur la surface externe du stent.

6. Stent utilisé dans une lumière corporelle selon la revendication 4 ou 5, dans lequel la forme de la fente de charge de médicament (3) est circulaire, rectangulaire, ovale, losange ou d'autres formes irrégulières.

7. Stent utilisé dans une lumière corporelle selon la revendication 4, dans lequel le nombre de fentes de charge de médicament (3) sur une entretoise de stent (1) est de 0 à 10, et les fentes de charge de médicament (3) sont réparties sur la une entretoise de stent (1) et espacées les unes des autres si le nombre de fentes de charge de médicament (3) est supérieur à un.

8. Stent utilisé dans une lumière corporelle selon la revendication 4 ou 5, dans lequel la forme transversale de la fente de charge de médicament (3) est rectangulaire, trapézoïdale avec un côté supérieur plus long et un côté inférieur plus court, une forme de U, une forme de V ou d'autres formes irrégulières.

9. Stent utilisé dans une lumière corporelle selon la revendication 4 ou 5, dans lequel la largeur de la fente de charge de médicament (3) est dans la plage de 0,021 mm à 0,085 mm.

10. Stent utilisé dans une lumière corporelle selon la revendication 4 ou 5, dans lequel la largeur de la fente de charge de médicament (3) est dans la plage de 0,030 à 0,070 mm.

11. Stent utilisé dans une lumière corporelle selon la revendication 4 ou 5, dans lequel la profondeur de la fente de charge de médicament (3) est dans la plage de 0,021 mm à 0,080 mm.
